(19) Europäisches Patentamt
European Patent Office
Office européen des brevets

(11) Publication number: **0 371 698 A1**

# (12) EUROPEAN PATENT APPLICATION

(21) Application number: **89312175.6**

(51) Int. Cl.[5]: **C07C 275/28, C07C 273/18**

(22) Date of filing: **23.11.89**

(30) Priority: **30.11.88 US 277837**

(43) Date of publication of application: **06.06.90 Bulletin 90/23**

(84) Designated Contracting States: **AT BE CH DE ES FR GB GR IT LI NL SE**

(71) Applicant: **ARCO CHEMICAL TECHNOLOGY INC.**
**3 Christina Centre Suite 902 201 N Walnut Street**
**Wilmington Delaware 19801(US)**

(72) Inventor: **Kesling, Haven S., Jr.**
**248 Friendship Road**
**Drexel Hill, PA 19026(US)**
Inventor: **Shawl, Edward T.**
**208 Martroy Lane**
**Wallingford, PA 19086(US)**
Inventor: **Zajacek, John G.**
**669 Clovelly Road**
**Devon, PA 19333(US)**

(74) Representative: **Cropp, John Anthony David et al**
**MATHYS & SQUIRE 10 Fleet Street**
**London, EC4Y 1AY(GB)**

## (54) Process for the preparation of methylene diphenylene bis (dialkyl-ureas) and polymethylene polyphenylene poly (dialkyl-ureas).

(57) Methylene diphenylene bis (dialkylureas) or polymethylene polyphenylene poly (dialkylureas) are prepared by reacting a methylene diphenylene diamine or a polymethylene polyphenylene polyamine with isocyanic acid (HNCO) to convert the amino groups of the amines to urea groups ($-NHCONH_2$) to give a bis urea or polyurea which is then reacted with a dialkyl amine having from 1 to 8 carbon atoms to produce the desired bis (dialkylurea) or poly (dialkylurea).

EP 0 371 698 A1

# PROCESS FOR THE PREPARATION OF METHYLENE DIPHENYLENE BIS (DIALKYL UREAS)AND POLY-METHYLENE POLYPHENYLENE POLY (DIALKYL-UREAS)

## FIELD OF THE INVENTION

The present invention relates to a process for the preparation of methylene diphenylene bis (dialkyl ureas) and the higher polymethylene polyphenylene poly (dialkylurea) homologs thereof which comprises reacting a methylene diphenylene diamine or a polymethylene polyphenylene polyamine with isocyanic acid to form a methylene diphenylene bis urea or a polymethylene polyphenylene polyurea which is then reacted with a dialkyl amine, such as diethylamine, to give the dialkyl urea products, which are useful for the preparation of organic isocyanates as well as for agricultural applications.

## BACKGROUND OF THE INVENTION

A number of processes have been reported for the preparation of mono- and disubstituted ureas and amines as is the preparation of, for example, the bis (diethylurea) of toluene by reacting toluene-2, 4-diisocyanate with diethylamine described in Japanese Kokai 76/149,400.

An article by N.A. Ivanov entitled "Synthesis of Substituted Ureas and Thioureas and their Thermal Stability", Chemistry Department of the Kalinin Agricultural Institute, describes the synthesis of thioureas, monosubstituted ureas and toluene-2, 4-diamines.

German Democratic Republic Industrial Patent No. 228,544 related to the production of acyl isocyanates describes the synthesis of 1, 1-diacyl-3, 3-dialkylureas from, for example 1, 1-dimethylurea.

Czechoslovakian Patent No. 200,441 discloses a method for the preparation of aminophenylurea by reacting phenylenediamine with one mole of cyanic acid in the presence of sodium or potassium cyanate.

An article of Y. Shimonura et al entitled "Reactions of Isocyanic Acid with Various Reagents", Fukui Daigaku Kogakuba Kenkyu Hokoku, Vol. 31, No. 2, pp 115, 1983 describes the reaction of 2-cyanoethylamine with isocyanic acid to give 2-cyanoethylurea. The synthesis of isocyanic acid from cyanuric acid by thermal decomposition is also set forth.

Applicants are not aware of any truly pertinent prior art that is deemed to be anticipatory or suggestive of the concept of the present invention.

## SUMMARY OF THE INVENTION

This invention relates to a novel method for the preparation of methylene diphenylene bis (dialkyureas) including the higher homologs thereof,the polymethyene polyphenylene poly (dialkylureas) which may be used in agricultural applications as insecticides and herbicides or further processed to useful isocyanate products. Methylene diphenylene diamines and/or polymethylene polyphenylene polyamines are reacted with isocyanic acid (HNCO) to convert the amino groups of said compounds to urea groups ($-NHCONH_2$) which urea compounds are then reacted with a dialkylamine to produce the desired methylene diphenylene bis (dialkylureas) or the polymethylene polyphenylene poly (dialkylureas).

The primary object of the present invention is to provide an improved process for the preparation of methylene diphenylene bis (dialkylureas) and the higher polymethylene polyphenylene poly (dialkyurea) homologs thereof in high yield and high conversion of reactants.

It is another object of this invention to provide an improved reaction system for the conversion of methylene diphenylene diamines and polymethylene polyphenylene polyamines to the corresponding urea compounds.

These and other objects and advantages of this invention will become apparent from the description of the invention which follows, and from the claims.

## DETAILED DESCRIPTION OF THE INVENTION

In accordance with this invention, methylene diphenylene bis (dialkylureas) having the general formula

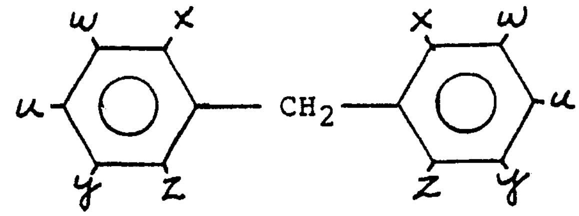

or the higher homologs thereof having the following structural formula

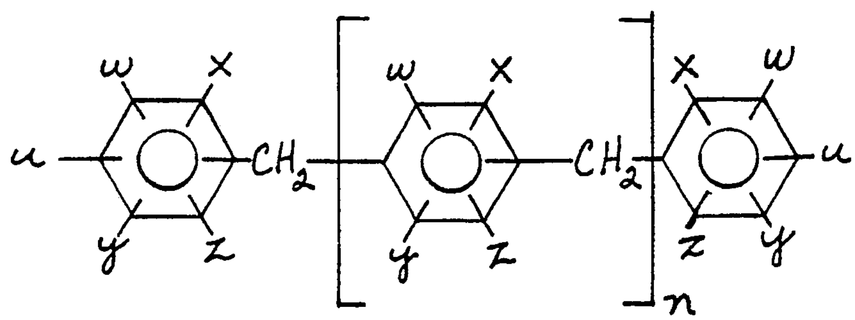

are produced wherein n equals an integer of from 1 to 8 and at least one of the substituents u, w, x, y and z on the ring is a dialkylureido group (-NHCONRR′) and the other substituents which may be different on the ring, are hydrogen, a 1 to 6 carbon alkyl group, a halogen, an ether group or a nitro group, R and R′ which may be the same or different are an alkyl group having independently from 1 to 8 carbon atoms which comprises reacting a methylene diphenylene diamine having the formula

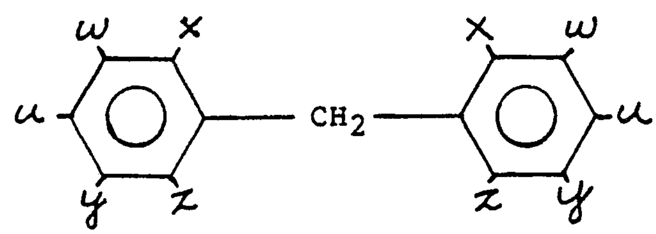

or a polymethylene polyphenylene polyamine having the formula

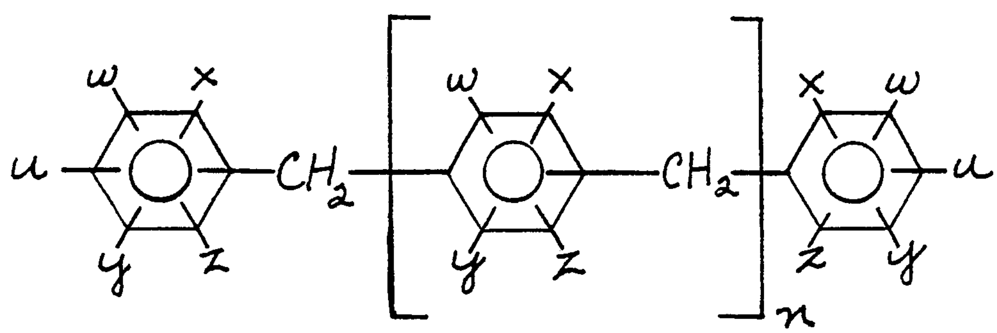

wherein n equals an integer of from 1 to 8 and at least one of the substituents u, w, x, y and z on the ring is a amino group with isocyanic acid at a temperature of from about -30°C to about 200°C preferably from about -10°C to about 150°C in the presence of a solvent or mixture of solvents which are stable and substantial chemically inert to the components of the reaction system, to convert the amino groups of the methylene diphenylene diamine or polymethylene polyphenylene polyamine to urea groups ($-NHCONH_2$) to produce methylene diphenylene bis urea or a polymethylene polyphenylene polyurea, and then reacting the

bis urea or polyurea thus produced with a dialkyl amine having from 1 to 8 carbon atoms in the alkyl group at a temperature of from about 50 °C to about 200 °C, preferably from about 90 °C to 150 °C in the presence of a solvent or mixture of solvents which are also stable and substantially chemically inert to the components of the reaction system and the desired methylene diphenylene bis (dialkylurea) or polymethylene polyphenylene poly (dialkylurea) product recovered.

The isocyanic acid employed in the process of the present invention may be produced or generated by known methods such as the pyrolysis of urea or cyanuric acid, reaction of cyanate salts such as sodium, potassium or silver cyanate and the like with an acid such as acetic or hydrochloric acid and the like. The isocyanic acid may be generated and used in situ, or it may be distilled away from its source and used in the process of the invention as a gas or dissolved in an appropriate solvent.

In the present process the molar ratio of the ($-NH_2$) groups of the methylene diphenylene diamines or polymethylene polyphenylene polyamines to isocyanic acid is generally one to one. However, an excess of isocyanic acid of up to about about 50% may be employed. Alternatively, an excess of up to about 30% ($-NH_2$) groups may be used and any unreacted or partially reacted diamines or polyamines separated from the bis urea produced and recycled. In the second part of the reaction system of the instant invention the molar ratio of the dialkylamine reactant, such as dimethylamine, to the urea groups ($-NHCONH_2$) is generally one to one. However, an excess of dialkylamine of from about 10% to a ten-fold excess may advantageously be employed to drive the reaction to completion. Unreacted dialkylamine may be easily recovered by distillation for recycle in the reaction.

The dialkylamines or mixtures thereof which may be employed in the process of the invention conform to the general formula $R'R''NH$ wherein $R'$ and $R''$ which may be the same or different are alkyl groups having independently from 1 to 8 carbon atoms. Representative dialkylamines include, for example, dimethylamine, diethylamine, methylethylamine, diisopropylamine, dicyclohexylamine, dibutylamine, and the like.

Solvents or mixtures of solvents which are stable and substantially chemically inert to the components of the reaction system are employed in the process steps of the present invention. Suitable solvents which may be employed include, for example, the aromatic hydrocarbons such as benzene, toluene, xylene, tetrahydronaphthalene as well as higher alkyl-substituted aromatic hydrocarbons; alkanes and substituted alkanes as well as cycloalkanes having from 5 to 20 carbon atoms such as, for example, n-hexane, n-heptane, octane, nonane, cyclohexane, dodecane, octadecane, 2-methylhexane, 2-ethylhexane, methylcyclohexane, cyclopentane and the like; halogenated or nitrated aromatic or aliphatic hydrocarbons such as, for example, methylene chloride, chloroform, carbontetrachloride, 1,2-dichloroethane, chlorobenzene, trichloroethane, tetrachloroethane, dichlorobenzene, nitrobenzene, dinitrotoluenes and the like; aromatic or aliphatic ethers such as, for example, diphenylether and dibutylether and the like; tertiary amines such as, for example, pyridine, triethylamine, N-methylpyrolidone and the like. Certain ketones, esters, alcohols as well as water and highly polar solvents, such as sulfolane, dimethylsulfoxide, ethylene carbonate or propylene carbonate may also be used. It is not necessary that the methylene diphenylene diamines or polymethylene polyphenylene polamines, the reaction intermediates such as the bis ureas or poly ureas or the reaction products be completely miscible with the solvents at the concentrations employed. Advantage may be taken of differing solubilities of the reagents, intermediates and reaction products in the various solvents or mixture of solvents to separate the reaction components or to drive the reaction to increased product. The same solvent or mixture of solvents may be used throughout the reaction system or different solvents or solvent mixtures used in different steps of the process.

The intermediate bis urea or polyurea obtained by the reaction of an methylene diphenylene diamine or polymethylene polyphenylene polyamine with isocyanic acid may, if desirable, be isolated from the reaction system and further processed according to the process of the invention or it may simply be carried forward as a solution or a slurry without isolation for completion of the process. In appropriate solvents, the intermediate bis urea or polyurea may be essensially insoluble and the urea easily separated from unreacted methylene diphenylene amine or polyamine or partly reacted amine urea by-products by conventional techniques such as filtration, centrifugation, and the like. The separated bis urea, polyurea or the crude intermediate reaction product may be reacted with a dialkyl amine to produce the desired urea product. The dialkyl amine reactant may be added as a gas, a liquid, a solid, or as a solution in solvent. Alternatively, the dialkyl amine may be added to the reactor along with the methylene diphenylene diamine or polymethylene polyphenylene polyamine. Ammonia generated in this part of the reaction is removed by any convenient means.

The process of the present invention may be carried out as a batch process in the same reactor employed for the first part of the reaction or as indicated hereinabove the intermediate separated and a separate reactor employed. The process may also be carried out on a semi-continuous or continuous basis

and the order of addition of the materials varied to suit the particular apparatus employed.

The reactions of the present process may be carried out in any suitable reactor which is equipped with a means for temperature control and agitation. A general procedure for carrying out the process is to charge the diamine or polyamine together with a solvent into the reaction vessel. The isocyanic acid is introduced into the reactor as a gas, optionally diluted with an inert gas, as a liquid, or as a solution in an appropriate solvent. Alternatively, the isocyanic acid can be charged to the reactor first together with a solvent and the diamine or polyamine then added as a liquid, a solid, a solution in suitable solvent or as a slurry in a suitable inert liquid. The reaction vessel is heated or cooled as necessary to provide the desired reaction temperature for the appropriate period. Heating and/or cooling means may be employed interior or exterior of the reaction vessel to maintain temperature within desired ranges. The desired reaction product may be recovered by standard filtration and/or distillation procedure.

In the process of the present invention, the reaction of the methylene diphenylene diamines or polymethylene polyphenylene polyamines with isocyanic acid will proceed at temperatures of from about -30° C to about 200° C, preferably from about -10° C to about 150° C. Reaction time is dependent on the temperature but will generally range between about two minutes to several hours. The reaction of the intermediate bis urea or polyurea and the dialkyl amines will proceed at temperatures of from about 50° C to about 200° C, preferably from about 90° C to about 150° C. The reaction time depends on temperature but will generally range between about 30 minutes to about 8 hours.

The process of the present invention is generally carried out at atmospheric pressure or the autogenous pressure of the reaction system, although higher pressures may be employed at the higher reaction temperatures or when the reaction temperature is above the boiling point of the solvent or dialkyl amine employed. Although subatmospheric pressure may be used there is no apparent value in employing same.

The following examples are provided to illustrate the invention in accordance with the principles of this invention and include particular features of the invention. However, the examples are not to be construed as limiting the invention in any way, it being understood that numerous variations are possible without departing from the spirit and scope of the invention.

## EXAMPLE 1

A mixture of 2.0g (10 mmoles) of 4,4′-methylene diphenylene diamine in 130g o-xylene was charged to a 250 ml, 3 neck round bottom flask equipped with a mechanical stirrer, condenser and thermometer. This mixture was heated to 130° C and the resulting solution was stirred while 15g of a 7.0 weight percent solution of isocyanic acid in o-xylene containing 1.05g (24 mmoles) of isocyanic acid was added over 10 minutes. Solids began to precipitate immediately on addition of the isocyanic acid solution. After 20 min. additional mixing, the solids were collected by filtration. Analysis of the filtrate and the recovered solids by high pressure liquid chromatography (HPLC) showed 96% conversion of the methylene diphenylene diamine with 98% selectivity to methylene diphenylene bis urea. The solids were returned to the reaction flask, suspended in 100g fresh o-xylene and 2.5g diethylamine (34 mmoles) was added and the mixture was heated for 2 hours. The initial temperature was 100° C. but as the diethylamine was consumed, the temperature rose to 135° C and the solids dissolved. Anaysis of the product by HPLC showed essentially quantitative conversion of the bis urea with 90% selectivity to methylene diphenylene bis (diethylurea). The product was isolated by distilling the solvent and unreacted diethylamine overhead using a rotary evaporator. The solid recovered, 3.7g, was analyzed by HPLC as 90% methylene diphenylene bis (diethylurea) and corresponded to an 85% overall yield of the bis (diethylurea) based on starting diamine.

## EXAMPLE 2

A 250 ml, 3 neck, round bottom flask equipped with a mechanical stirrer, condenser, and thermometer was used. Polymethylene polyphenylene polyamine, 2g, made by condensing aniline with formaldehyde using methods described in the literature (Kirk-Othmer, Encyclopedia of Chemical Technology, 3rd Edition, Vol. 2, pp 342-343, John Wiley and Sons, N.Y. 1978) in 100g mixed xylenes was treated with 25g of 5.0 weight percent solution of isocyanic acid in mixed xylenes at 80° C. An oil formed as the isocyanic acid was added. After 30 min. mixing, the excess isocyanic acid was distilled out and then 3g of diethylamine was added. The mixture was heated to a maximum of 140° C over three hours. Analysis of the product by a

combination of HPLC and nuclear magnetic resonance showed that a mixture of polymethylene polyphenylene poly (diethylureas) had been made with an approximate yield of 85% based on starting polyamine.

## EXAMPLE 3

Isocyanic acid, generated by the reaction of sodium cyanate with hydrogen chloride, was distilled away from the sodium cyanate and carried as a gas diluted with nitrogen into a 250 ml 2 neck round bottom flask equipped with a magnetic stirrer, condenser, and gas inlet tube containing 1.20g of 4,4′-methylene diphenylene diamine in 130g o-dichlorobenzene. The reaction was held at -10° C by a refrigerated bath. Solids precipitated in the flask on addition of the isocyanic acid. After an excess of isocyanic acid has been added. the reaction was warmed to room temperature and stirred for 30 minutes. The solids formed were collected by filtration, transferred to a pressure vessel, and suspended in 100 ml mixed xylenes. Dimethylamine, 1g, was charged to the vessel which was then heated to 140° C under autogenous pressure for 4 hours. Analysis of the product by HPLC showed an overall yield of 90% of 4,4′-methylene diphenylene bis (dimethylurea).

## EXAMPLES 4 - 8

A number of runs were carried out according to the procedures of Example 2 employing various diamines, polyamines, dialkylamines, solvents and reaction temperatures . The product ureas were characterized by high pressure liquid chromatography (HPLC).

The reaction conditions and results are set forth in the Table below.

TABLE

| Example No. | Polyamine or Diamine (g.) | Solvent (g.) | HNCO-g. Solvent (g) | Temp. (°C) | Time | Dialkyl Amine (g.) | Temp. (°C) | Time | % yield on Polyamine or diamine |
|---|---|---|---|---|---|---|---|---|---|
| 4 | 4′,4′/2,4′ MDA 90/10 (1.22) | Diphenyl Ether (100) | 1.00 Diphenyl Ether (10) | 150 | 10 min | DEA (15) | 100 | 8 hrs | 80 |
| 5 | PMPPA (2.4) | Nitro-Benzene (50) | 5 (as gas) | 30 | 1 hr. | DMA (15) | 150 | 2 hrs | 82 |
| 6 | 4,4′-MDA (1.22) | 1,2-Dichloroethane (100) | 1.00,1,2-dichloroethane (10) | 50 | 1/2 hr. | DBA* (3) | 90 | 8 hrs | 77 |
| 7 | 4,4′-MDA (1.22) | Octadecane (100) | 1.00 Toluene (20) | 30 | 1 hr. | DIPA (4.2) | 130 | 3 hrs. | 89 |
| 8 | 4,4′-MDA (1.22) | o-Dichlorobenzene (100) | 0.70 (as gas) | 50 | 1 hr. | DEA (3) | 125 | 2 hrs | 82 |

* - Carried out under 100 psig pressure
MDA - Methylene diphenylene diamine
PMPPA - Polymethylene polyphenylene polyamine
DEA - Diethylamine
DBA - Dibutylamine
DIPA - Diisopropylamine
DMA - Dimethylamine

## Claims

1. A process for the preparation of methylene diphenylene bis (dialkylureas) having the formula

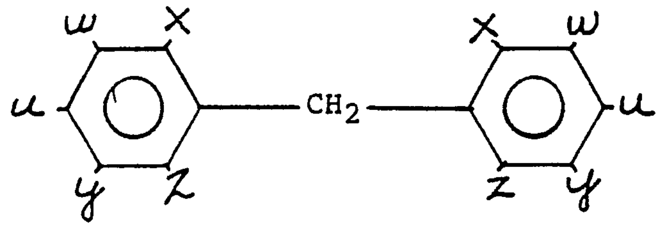

or the polymethylene polyphenylene poly (dialkyurea) homologs having the structural formula

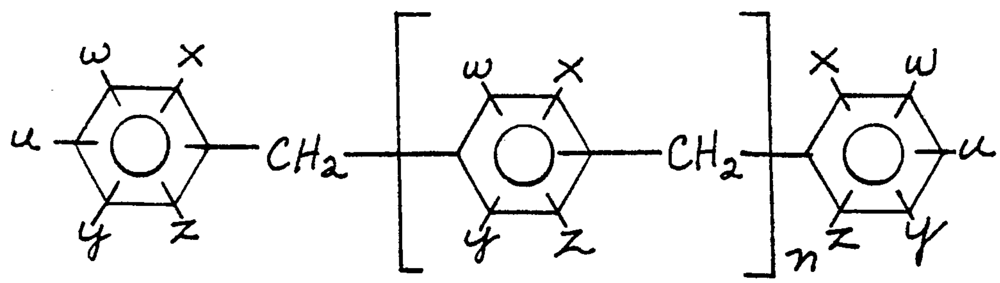

wherein at least one of the substituents u, w, x, y and z on the ring is a dialkylureido (-NHCONRR′) group and the other substituents which may be different on the ring, are hydrogen, a 1 to 6 carbon alkyl group, a halogen, an ether group or a nitro group, R and R′ which may be the same or different are an alkyl group having independently from 1 to 8 carbon atoms and n equals an integer of from 1 to 8, which comprises reacting a methylene diphenylene diamine having the formula

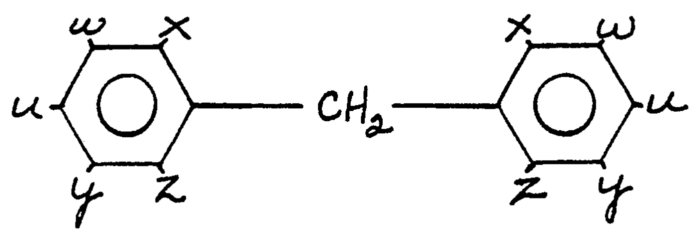

or a polymethylene polyphenylene polyamine having the formula

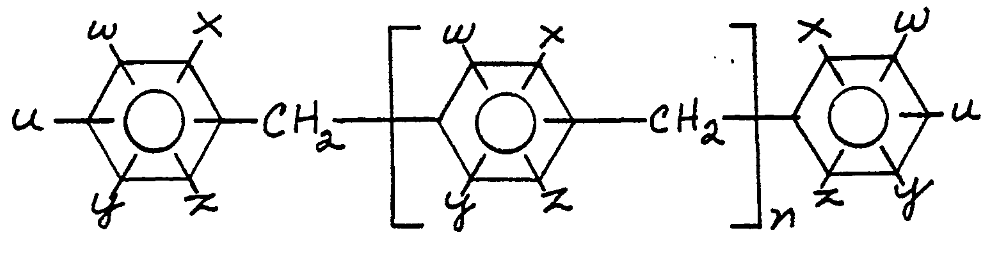

wherein at least one of the substituents u, w, x, y and z is amino group and n is an integer of from 1 to 8, with isocyanic acid at a temperature of from about -30°C to about 200°C in the presence of an essentially inert solvent or mixture of solvents to convert the amino groups of the methylene diphenylene diamine or

polymethylene polyphenylene polyamine to urea ($-NHCONH_2$) groups to produce a methylene diphenylene bis urea or a polymethylene polyphenylene polyurea and then reacting said methylene diphenylene bis urea or polymethylene polyphenylene polyurea with a dialkylamine having from 1 to 8 carbon atoms in the alkyl group at a temperature of from about 50°C to about 200°C in an essentially inert solvent, and recovering the desired methylene diphenylene bis (dialkylurea) or polymethylene polyphenylene poly (dialkylurea).

2. A process according to Claim 1 wherein the temperature of reacting the diamine or polyamine with isocyanic acid is in the range of from about -10°C to about 150°C.

3. A process according to Claim 1 wherein the temperature of reacting said bis urea or polyurea with a dialkylamine is in the range of from about 90°C to about 150°C.

4. A process according to Claim 1 wherein the methylene diphenylene diamine is 4,4′- or 2,4′-methylene diphenylene diamine or mixture thereof.

5. A process according to Claim 1 wherein the inert solvents are selected from the group consisting of xylenes, o-dichlorobenzene, 1,2-dichlorethane, diphenylether, nitrobenzene and octadecane.

6. A process according to Claim 1 wherein the dialkylamine reactant is selected from the group consisting of dimethylamine, diethylamine, diisopropylamine and dibutylamine

7. A process according to Claim 6 wherein the dialkyl amine is dimethylamine.

8. A process according to Claim 6 wherein the dialkylamine is diethylamine.

9. A process for the preparation of a methylene diphenylene bis (dialkylurea) having the formula

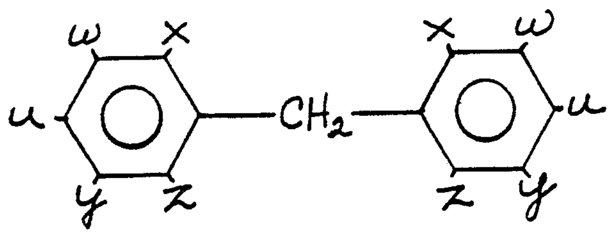

wherein at least one of the substituents u,w,x,y and z on the ring is a dialkylureido ($-NHCONRR'$) group and the other substituents which may be diffferent on the ring are hydrogen, a 1 to 6 carbon alkyl group, a halogen, and ether group or a or a nitro group which comprises reacting a methylene diphenylene diamine having the formula

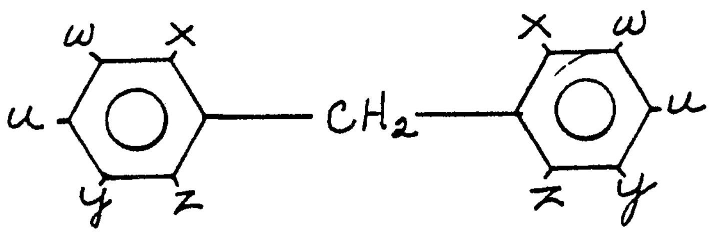

wherein at least one of the substituents u, w, x, y and z is an amino group, with isocyanic acid at a temperature of from about -10° to about 150°C in an essentially inert solvent or mixture of solvents to convert the amino groups of the methylene diphenylene diamine to urea($-NHCONH_2$)groups to produce a methylene diphenylene bis urea and then reacting said bis urea with a dialkylamine having from 1 to 8 carbon atoms at a temperature of from about 90°C to about 150°C in an essentially inert solvent or mixture of solvents and recovering the desired methylene diphenylene bis (dialkylurea).

10. The process according to Claim 9 wherein the methylene diphenylene diamine is 4,4′ methylene diphenylene diamine and the dialkylamine is diethylamine.

European Patent Office

# EUROPEAN SEARCH REPORT

Application number

EP 89312175.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁵) |
|---|---|---|---|
| X | EP - A2 - 0 041 145 (BASF) * Claims 1,6; pages 4-5, line 27 * -- | 1-5,9, 10 | C 07 C 275/28<br>C 07 C 273/18 |
| D,X | PATENT ABSTRACTS OF JAPAN, unexamined applications, C section, vol. 1, no. 29, March 28, 1977 THE PATENT OFFICE JAPANESE GOVERNMENT page 1628 C 76 * Kokai-no. 51-149 400 (HITACHI SEISAKUSHO) * ---- | 1,9 | |

| TECHNICAL FIELDS SEARCHED (Int. Cl.⁵) |
|---|
| C 07 C 275/00<br>C 07 C 273/00 |

The present search report has been drawn up for all claims

| Place of search | Date of completion of the search | Examiner |
|---|---|---|
| VIENNA | 01-03-1990 | REIF |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document